(19) **Europäisches Patentamt**

**European Patent Office**

**Office européen des brevets**

(11) **EP 1 512 973 A1**

(12) **EUROPEAN PATENT APPLICATION**

(43) Date of publication:
09.03.2005 Bulletin 2005/10

(51) Int Cl.⁷: **G01N 33/68**, **C07K 14/47**

(21) Application number: **03077783.3**

(22) Date of filing: **04.09.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **CEDI Diagnostics B.V.**
**8219 PH Lelystad (NL)**

(72) Inventors:
• **Steenbergen, Jolanda**
**8383 EJ Nijensleek (NL)**

• **Heerkens, Sijmie**
**8231 JS Lelystad (NL)**
• **Schielen, Wilhelmus , Joseph, Gerardus**
**8245 CN Lelystad (NL)**

(74) Representative:
**Prins, Adrianus Willem, Mr. Ir. et al**
**Vereenigde,**
**Nieuwe Parklaan 97**
**2587 BN Den Haag (NL)**

(54) **Methods and kits for detection of prion diseases**

(57)     The invention relates to diagnostic methods and kits for detecting transmissible spongiform encephalopathies (TSEs) such as BSE, scrapie, chronic wasting disease and related diseases in animals and humans.

The invention provides a method for determining whether an aberrant prion protein is present or absent in a mammalian sample comprising the steps of preparing in an extraction buffer that comprises a surfactant and a detergent a homogenate from said sample, incubating said homogenate in diluted extraction buffer with a protease, incubating with a chaotropic agent and at least one washing buffer and detecting said aberrant prion protein.

**EP 1 512 973 A1**

Printed by Jouve, 75001 PARIS (FR)

**Description**

The invention relates to diagnostic methods and kits for detecting transmissible spongiform encephalopathies (TSEs) such as BSE, scrapie, chronic wasting disease and related diseases in animals and humans.

Bovine spongiform encephalopathy (BSE or mad cow disease) of cattle and scrapie of sheep are fatal, non-inflammatory neurodegenerative diseases caused by prions and are characterized by a long incubation period. In humans Creutzfeldt-Jakob disease (CJD), Gerstmann-Sträussler-Scheinker syndrome (GSS), fatal familial insomnia and kuru belong to this category of TSEs.

Although scrapie, the prototype of the family of TSEs in sheep and goats has been known for over 200 years (Pattison, 1988) and has been diagnosed world-wide (with the exception of New Zealand and Australia), it is only since 1986 that BSE has been described in cattle in the UK. By January 2003, there had been more than 190.000 confirmed cases of BSE in Great Britain and there may exist a great number of cases of not yet overt ("silent") BSE. BSE probably emerged because scrapie-contaminated sheep offal had been included in cattle feeding-stuff via meat and bone meal and newly infected cattle material was then recycled (Wilesmith et al., 1991). This mechanism is quite plausible since ovine scrapie could be transmitted experimentally to several animal species, including cattle (Hourrigan, 1990; Gibbs, 1990). Alternatively, recycling of offal from a rare case of spontaneous BSE for cattle feedstuff could also have led to the BSE epidemic. Moreover, the number of cattle in the UK with BSE reported annually is declining after the ban on feeding meat and bone meal in 1988.

Brain homogenates from cows with BSE produce after inoculation of mice a characteristic pattern of brain lesions in mice. Also characteristic incubation periods in inbred lines of mice are seen. This is identical to the pattern elicited by brain tissue from individuals who have died from new-variant Creutzfeldt-Jakob disease (nvCJD; Bruce, 1997). The conclusion is that the BSE agent is identical to the nvCJD agent. Up to now, this variant has caused the death of 129 young Britons and 7 Frenchmen (Will et al, 1996; info: CJD Statistics per 6 May 2003, Internet).

There is also concern that the BSE strain that seems to be transmissible to humans may have infected sheep, where it could produce a disease hardly distinguishable from scrapie. When its ominous strain-specific properties are maintained across the species barrier, sheep BSE may be a threat to human health, although scrapie by itself seems not to transmit to humans. Indeed, BSE agent has been transmitted experimentally to sheep by the oral route (Foster et al., 1993) and thus could have the potential to infect sheep under field conditions. With the exception of a bioassay in mice, no diagnostic method is available to discriminate between BSE and scrapie in sheep at present.

Thus far, the only known component of the infectious prion is an abnormal, disease-causing isoform of the "normal" prion protein (PrP) called Prp$^{Sc}$ or aberrant prion protein (the terms will be used interchangeably herein). PrP, or normal prion protein, is ubiquitous in mammalian cells in a benign, cellular conformation (PrP$^{C}$) and is encoded within a single exon as a protein of about 250 amino acid residues. The PrP gene has been cloned and sequenced from a variety of species and there is a high degree of structural and organisational homology between mammalian PrP sequences (Schatzl et al., 1995). PrPs in many mammals have a 22-24 residues long N-terminal signal sequence as well as a 22-24 residues long C-terminal signal sequence for attachment of a GPI-anchor. This glycosyl-phosphatidylinositol linkage is a fairly common means of anchoring proteins to membranes of eukaryotic cells. Further structural characteristics of the mature protein (of 206-210 amino acid residues) are one disulfide bond and two sites for Asn-linked glycosylation.

PrP$^{Sc}$ originates from the normal cellular isoform (PrP$^{C}$) by a post-translational process since the amino acid sequence of PrP$^{Sc}$ is identical to that predicted from cDNA or genomic nucleic acid sequences. Glycosylation patterns are also identical between PrP$^{c}$ and PrP$^{Sc}$. Moreover, Caughey & Raymond (1991) demonstrated that PrP$^{Sc}$ is made from a cell surface precursor that is identical to the normal PrP. PrP$^{Sc}$ differs from the normal, membrane bound cellular prion protein by its relative protease resistance. Treatment with proteinase K (PK) for instance, results in complete proteolysis of PrP$^{C}$ whereas in PrP$^{Sc}$ the N-terminal part is removed before the amino acid at position 90 (human numeration). The protease-resistant core left is designated PrP27-30 after its electrophoretic behaviour in SDS-PAGE as a protein molecule with $M_r$ = 27-30 kDa, and this molecular species retains full infectivity.

Further distinguishing features of PrP$^{Sc}$ are its thermal stability, a strong tendency to aggregate and insolubility in non-denaturing detergents, apparently connected with a different molecular structure. All attempts to identify a post-translational chemical modification that features in the conversion of PrP$^{C}$ into PrP$^{Sc}$ have been unsuccessful.

The lack of a molecular explanation for the observed differences between PrP$^{Sc}$ and PrP$^{C}$ led to the proposal that they must differ in conformation. Indeed, Fourier transform infrared spectroscopy detected a content of 43% of β-sheet and 30% of α-helix structure for purified hamster PrP$^{Sc}$ and an even higher β-sheet content of 54% for PrP27-30. On the other hand a low content of β-sheet structure and a high α-helix content of 42% was found in PrP$^{C}$, suggesting differences in secondary structure between the aberrant and normal forms of PrP (Pan et al., 1993).

Due to its better solubility and the availability of recombinant forms of PrP$^{C}$, the three-dimensional structure of mouse PrP(121-231), involving three α-helices and a short antiparallel β-sheet, could be established by NMR (Riek et al. 1996). In the mature murine PrP$^{C}$(23-231), this segment seems to have the same fold (Riek et al., 1997). Also the spatial structure of recombinant hamster PrP(29-231) has been examined (Donne et al., 1997).

A species barrier for prion infection has been convincingly documented and found to vary widely depending on the pair of species involved and the direction of transmission. A structural basis for this species barrier is theoretically related to part or all of the amino acid replacements between the PrP of a given pair of species (Billeter et al., 1997).

Within species, genetic polymorphism in the PrP gene has been found for example with mice, humans and sheep. In sheep amino acid substitutions in PrP at a few different positions were found to correlate with different predispositions for the development of scrapie (Laplanche et al., 1993; Hunter et al., 1994; Belt et al., 1995; Bossers et al., 1996).

Studies of scrapie in goats and mice demonstrated reproducible variations in disease phenotype (length of incubation times and pattern of vacuolation) with the passage of prions in genetically inbred hosts (Bruce & Fraser, 1991). The distinct varieties or isolates of prions were called "strains". Safar et al. (1998) made plausible that the biological properties of prion strains are enciphered in the conformation of $PrP^{Sc}$ and that strains represent different conformations of $PrP^{Sc}$ molecules. Infection of Syrian hamsters with eight different hamster-adapted scrapie isolates produced $PrP^{Sc}$ molecular species, which isolated from brains in the terminal stages of disease, differed with respect to protease resistance and unfolding behavior under denaturing conditions. Differences in glycosylation have also been proposed as "strain-specific" properties (Collinge et al., 1996).

Animals and humans lack a TSE disease-specific immune response and TSE diagnosis is based mainly on histopathological examination, which relies on the observation of neuronal degeneration, grey matter vacuolation (the spongiform change) and astrocytosis. A distinguishing feature of TSE is the accumulation of aberrant protein ($PrP^{Sc}$) in the brain under continuing biosynthesis of the normal cellular $PrP^{C}$. Species differences exist however, since the relative accumulation of $PrP^{Sc}$ in brains of hamster and mouse is approximately 10x as high as in the ruminant. Unlike the normal $PrP^{C}$, $PrP^{Sc}$ can aggregate into amyloid-like fibrils and plaques and is a major component of brain fractions enriched for scrapie activity. Therefore, a more specific diagnosis of TSE is detection of $PrP^{Sc}$ either in situ e.g. by immunohistochemistry or in tissue homogenates e.g. by Western blot.

Several poly- or monoclonal antibodies to PrP have been described. The antisera were raised in mice, hamsters, rabbits and PrP null mice and as immunogens, peptides (as linear epitopes), purified and formic acid treated $PrP^{Sc}$ from mice, hamster or sheep and recombinant PrP are being used. However, except for one case (Korth et al., 1997), no antibodies have been developed which can discriminate between native forms of $PrP^{C}$ and $PrP^{Sc}$, and such antibodies cannot likely discern the difference between prion strains.

By Western blotting or immunohistochemistry $PrP^{Sc}$ could be detected in sheep in brain, spleen, tonsil or lymph node material and even in a preclinical stage of scrapie (Schreuder et al., 1998). However, in BSE infected cattle $PrP^{Sc}$ could not be detected outside the central nervous system, not even when clinical symptoms were present.

The intriguing mechanism of prion replication is not fully understood. According to the prevailing theory, the infectious $PrP^{Sc}$ acts as a template in the replication of nascent $PrP^{Sc}$ molecules. In other words $PrP^{Sc}$ imposes its own conformation upon the cellular form $PrP^{C}$ or an intermediate form. A thus far unknown protein X may function as a molecular chaperone in this formation of $PrP^{Sc}$ (Prusiner et al., 1998).

Because of the connection between BSE and the nvCJD, and the possible transfer of BSE to other species including sheep, there is a need to monitor for example slaughter cattle and sheep for the presence of aberrant prion protein before the meat and meat products enter the human and animal food chain or into pharmaceuticals prepared for human and animal use. Mass screening of sheep and cattle should also be of help in view of eradication programmes of scrapie and BSE. Moreover, human blood and blood products may form a health threat on account of possible contamination with blood of CJD patients and the recent occurrence of the nvCJD. For these monitoring purposes a detection method for aberrant prion protein has to be developed which should be fast, sensitive, reliable and simple.

Multiple TSE detection methods have been disclosed and a review is provided in our co-pending patent application WO 00/48003 (as incorporated herein by reference).

Thus far, four commercial assays (for example the Swiss company Prionics Inc. and the Irish Company Enfer Scientific Ltd.) have been announced. However, these tests, although claiming high sensitivity in detecting the aberrant protein, and thus claiming to have a low number of false-negative results, suffer from the low specificity associated with the claimed high sensitivity. When using the above tests one therefore runs an increased risk of falsely identifying a negative sample as false-positive, thereby falsely identifying an animal as positive. For example, Switzerland slaughtered herds in which one or more cases of BSE had been confirmed. The "Swiss reference laboratory for animal TSE" examined the brains of these 1761 apparently healthy cattle by an immunohistochemical method for signs of BSE and six positive cases were detected. Also Prionics Inc. tested these 1761 cattle brains by their "BSE Western Test". Four positive outcomes were identical to the ones found by the reference laboratory, the other two were indicated as negative and moreover two other cattle were found positive by Western blotting. Thus a total of eight positive reactors were found, four of which overlapped. These eight were re-examined in the laboratory of Dr Kretzschmar (University of Göttingen) and in addition to the four undisputed cases, one of the two questionable cases identified by the reference laboratory could be confirmed (info: New Scientist, 1998, July 4 and Internet). Prionics for example scored 0.1% false-positives, indicating that in 1 of every thousand cases a sample causes a false-alarm due to false-positivity.

Tests scoring false-positive results (being in general not specific enough) have other consequences than tests scoring false-negative results (being in general not sensitive enough).

False-negative means that an in essence positive sample from a positive individual is scored negative, and thus is not suspected of having a TSE while in truth said individual is having a TSE. A false-negative diagnosis thus results in missing positive cases.

For humans, false-negative means that no diagnosis of TSE is made where said human actually has a TSE. This causes a wrong prognosis being established and wrong treatment being given, until a second test is done.

For animals, especially in those cases where slaughtered animals are tested, false-negative means that no diagnosis of TSE is made where said animal was actually infected and possibly capable of spreading the disease without having been noticed. Meat and other products from such a false-negative animal may contain aberrant prion protein. Such meat and meat products will be traded and eaten, and can thus be a source for further infection, notably of humans who even falsely trust that the animal has been tested well and the meat or meat product bears no risk.

False-positive means that an in essence negative sample from a negative individual is scored positive, and thus is at least suspected of having a TSE while in truth said individual is not having a TSE at all, but possibly another condition.

For humans, false-positive means that a false diagnosis of TSE is made, here again resulting in false prognosis, and in faulty treatment. If said individual is not treated well as a consequence of the mis-diagnosis, his or her possible other disease condition (the symptoms of which for example gave rise to the decision to test for TSE) receives no proper treatment.

For animals, false-positive means that a false diagnosis of TSE is made, however, since TSEs are notifiable diseases that in general are met with strict eradication measures, said animal must, at least in most Western countries be killed and destroyed. Furthermore, the herd from which said animals originated runs the same risk of being destroyed when the diagnosis is not corrected. For the slaughterhouse it might mean that special laborious decontamination actions have to be implemented which mean temporary interference of use of the facilities and thus considerable loss of productivity. Additionally, the country where said animal or herd is falsely diagnosed for having a case of TSE among its animals will be met with export restrictions. It goes without saying that, especially when said country has no (present) reported cases of TSE, such a false-positive diagnosis is highly detrimental for said countries position on foreign markets for animal products.

Understanding the above risks associated with false-negative or false-positive diagnoses becomes even more complicated when one understands that in general the level of false-positives scored by a diagnostic method or test is inversely related to the number of false-negatives scored by the same test. It is an old diagnostic truth that, in many instances, a very sensitive test (having low numbers of false-negatives) cannot be very specific (and thus has a relative high number of false-positives) and vice versa. However, and especially for mass screening tests that do not comprise histology or cytology, and wherein many samples need to be tested, tests having both high sensitivity and specificity are desired.

Our co-pending patent application WO 00/48003 discloses a method for reducing the risk of scoring a false-positive and/or false-negative test result. WO 00/48003 discloses the use of guanidine thiocyanate (gdnSCN) or a functional equivalent thereof for treating at least one sample derived from a mammal for reducing the risk of scoring a false-positive or false-negative test result in testing said sample for the presence or absence of aberrant prion protein.

Although, the application of the method as described in WO 00/48003 provides an important improvement in respect of the diagnostics of TSE further improvements are required. The demands from users (for example laboratories testing slaughter cattle) and official institutes like the European Union are increasing, for example there is an ongoing need for a method that reduces the risk of scoring a false-positive and/or false-negative test even further or a method that takes less time or a method that results in less waste material or, a method that comprises an internal control for each sample or combinations thereof. It is clear that improvements with regard to for example methods that are faster or methods that produce less waste preferably do not compromise the selectivity and/or specificity.

The present invention provides an improved method for detecting aberrant prion protein. Particular improvements over the present available methods are outlined below.

In a first embodiment, the invention provides a method for determining whether an aberrant prion protein is present or absent in a mammalian sample comprising the steps of preparing a homogenate from said sample in an extraction buffer that comprises a surfactant and a detergent, incubating said homogenate in diluted extraction buffer with a protease, incubating with a chaotropic agent and at least one washing buffer and detecting said aberrant prion protein.

In a more preferred embodiment, the method as described above further comprises applying said homogenate, at any desired phase in said method, to a carrier.

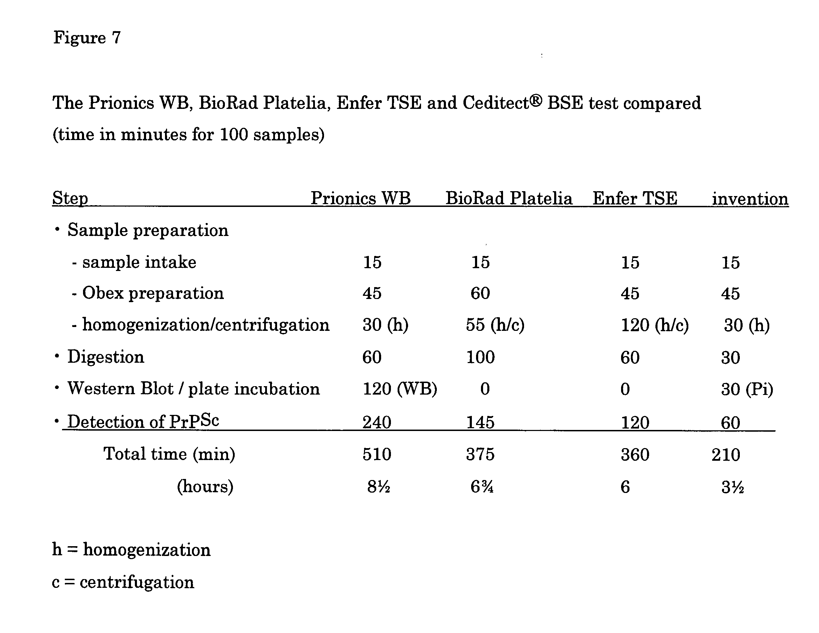

A comparison of the method according to the invention with 3 other tests (Prionics WB, BioRad Platelia and Enfer TSE; Figure 7) shows that a method according to the invention is performed almost in half the time needed for the prior art diagnostic methods. Hence, the invention provides a method for determining whether an aberrant prion protein is present or absent in a sample with a strongly reduced analysis time. Preferably the time to come to a complete analysis is less than 6 hours, more preferably, less than 5 hours, even more preferably less than 4 hours and most preferred the analysis is completed in approximately 3.5 hours. This is particularly advantageous for testing of slaughter animals. Samples are taken (early) in the morning, the results of the TSE diagnosis are then available late in the morning or early in the afternoon and the slaughter procedures are then be finished on the same day. Testing and slaughtering on the same days increases the efficiency and logistic in slaughterhouses.

Yet another advantage of a method according to the invention is the fact that the buffers used for homogenisation and protease treatment comprises the same components, albeit at a different concentration. The extraction buffer comprises a surfactant and a detergent and the buffer that is used to dilute the prepared sample is a diluted variant of this extraction buffer. This buffer system ensures acceptable protein extraction, protease treatment as well as binding of the protein to a carrier. Moreover, due to the fact that the buffers used for homogenisation and protease treatment comprises the same components, albeit at a different concentration, lengthy buffer exchange procedures and lengthy washing procedures are not required. Hence, a method according to the invention is performed in less time in comparison to other TSE detection methods.

The present inventors have performed multiple experiments in respect of the used extraction buffer (that comprises a surfactant and a detergent) and the effect of this buffer on subsequent protease treatment and binding to a carrier. Preferably, said extraction buffer that comprises a surfactant and a detergent comprises NaDOC as surfactant and Triton (X-100) as detergent. The inventors have performed multiple experiments with different dilutions and different buffers and a preferred embodiment is deduced from the following results. After a homogenate was prepared, a 1/30 dilution of the obtained homogenate in a 1/10 dilution of the extraction buffer resulted in the lowest obtained signal (i. e. the protease has performed the best; 47 kRLU = kilo relative light units). The use of an undiluted extraction buffer, 1/100 dilution and a 1/1000 dilution of the extraction buffer resulted in a signal of 51 kRLU, 88 kRLU and 263 kRLU respectively (the higher the amount of kRLUs the larger the amount of non-digested protein). Instead of diluted extraction buffer also PBS and PBST were tested in their suitability for protease treatment. The use of these buffers resulted in a signal of 251 kRLU for PBS and 102 kRLU for PBST.

Besides the fact that a 1/30 dilution of the homogenate in 1/10 diluted extraction buffer provides an acceptable amount of protease activity, this 1/10 diluted buffer also provides an acceptable binding of the proteins to a carrier and hence, in a preferred embodiment the present invention makes use of a 1/10 diluted extraction buffer for protease treatment and binding to a carrier.

Non-limiting examples of suitable surfactants are natrium deoxycholate (NaDOC), Amphomycine, dibutoline sulphate or calcium ducosate and nonlimiting examples of suitable detergents are Triton (for example Triton X-100), Nonoxynol, Ammonyx or Poloxalene. In a preferred embodiment the used surfactant is NaDOC and/or the used detergent is Triton (X-100) and even more preferably NaDOC and Triton (X-100) are both used in concentrations of 0.5%.

In another preferred embodiment, the used carrier is a filter and in an even more preferred embodiment, said filter is a PVDF filter. It is clear to a person skilled in the art that said PVDF filter must be activated, for example by soaking in or incubation with methanol, before a (treated, i.e. protease and/or chaotropic agent) sample is applied to said PVDF filter. Preferably, the (PVDF) filter is part of an ELISA-plate to facilitate easy handling (for example by using a vacuum manifold). More preferably, different parts of such a PVDF-filter (ELISA) plate are colour-coded to further increase the ease of handling. In a preferred embodiment, homogenates are prepared, transferred to a deep-well collection plate, diluted, transferred to an incubation plate and finally transferred to a PVDF-filter plate.

The ratio of sample to extraction buffer typically ranges from 1:10 (10% w/v) up to 1:5 (20% w/v). Provided that the concentration is between 10% and 20% (w/v) the method according to the invention accepts minute amounts of homogenate. In cases where only very small pieces/slices of a sample are available (down to 50 mg) a simple adjustment of the amount of extraction buffer is sufficient.

In principle, any method of homogenization (eg. MediFastH, FastH, UltraTurrax, Ribolyzer, Stomacher, Cotton cloth press, etc.) is allowed in a method according to the invention. Preferably, the homogenization method produces at maximum 0.40 µm particles with an acceptable amount of non-pipettable debris. For practical reasons MediFast, FastH and UltraTurrax based homogenization procedures are preferred.

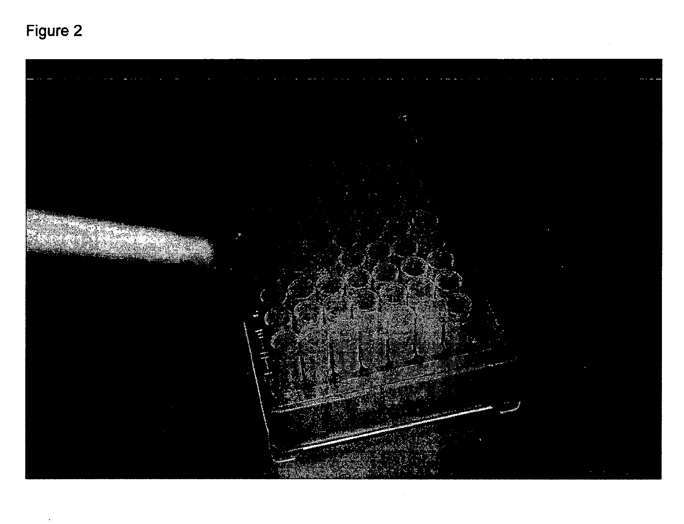

In a preferred embodiment, each homogenate is transferred, after homogenization, to (a unique position in) a deep-well collection plate. Collection of the produced homogenates in a collection plates facilitates easy and fast processing of the subsequent method steps. In an even more preferred embodiment, the deep-well collection plate has been colour-coded for further convenience (for example a green square printed around row A, as can be seen in Figure 2).

An example of an aberrant prion protein that is detected with the present invention is PrP$^{Sc}$ and hence the present invention provides a method for determining whether PrP$^{Sc}$ is present or absent in a mammalian sample or in other words the present invention provides a BSE-test and/or a scrapie test and/or a CWD test.

In a preferred embodiment, the invention provides a method for determining whether an aberrant prion protein is present or absent in a mammalian sample comprising the steps of preparing a homogenate from said sample with an extraction buffer that comprises a surfactant and a detergent, incubating said homogenate in diluted extraction buffer with a protease, incubating with a chaotropic agent and at least one washing buffer and detecting said aberrant prion protein, wherein said aberrant prion protein is detected in an immunoassay. The prior art discloses multiple examples of suitable immunoassays, for example Western Blot analysis, dot-blot methods, ELISA and many others. The method according to the invention preferably uses a modified dot-blot method as described herein. In short, a PVDF filter, optionally supported by another filter (for example a Whatman filter) is used in an ELISA plate from which the bottoms have been (partially) removed. This modified dot-blot method enables high-trough put analysis by for example using a vacuum manifold in multiple steps.

In a preferred embodiment, detection of said aberrant prion protein comprises the steps of incubating said carrier with blocking buffer, a compound capable of recognizing said aberrant prion protein and visualising said compound. More detailed steps of the detection method/steps are provided herein within the experimental part. Preferably, said blocking buffer comprises polyvinylalcohol (PVA) or polyvinylpyrrolidone (PVP) or PEG or different kinds of gelatines and even more preferably said blocking buffer further comprises bovine serum albumin (BSA) or defatted milkpowder (for example SkimMilk (SM) from Gibco or Elk from Campina or Profitar from Nutricia) or ovalbumin or casein. More preferably, said blocking buffer comprises PVA (for example PVA 30-70 kDa) and BSA (for example fractionV) in trisbuffer/tween (TBST) and even more preferably, said blocking buffer comprises 1% PVA and 0.5% BSA in TBST. However, other suitable combination are 0.5% SM and 2% PVA or 1% SM and 0.5% PVA or 1% SM and 2% PVA or 2% SM and 1% PVA. With the herein outlined procedure a person skilled in the art is very well capable of selecting other combinations of components that result in useful blocking buffers.

Surprisingly, the use of the above-described blocking buffers (for example 1% PVA and 0.5% BSA in TBST) reduces the risk of scoring a false-positive and/or false-negative test result. Hence, the invention provides a method for determining whether an aberrant prion protein is present which reduces the risk of identifying a false-positive or a false-negative.

In yet another preferred embodiment, the invention provides a method for determining whether an aberrant prion protein is present or absent in a mammalian sample comprising the steps of preparing a homogenate from said sample with an extraction buffer that comprises a surfactant and a detergent, incubating said homogenate in diluted extraction buffer with a protease, incubating with a chaotropic agent and at least one washing buffer and detecting said aberrant prion protein, wherein said chaotropic agent is guanidine thiocyanate. Even more preferably, the guanidine thiocyanate is applied in a concentration of approximately 4M. However, other chaotropic agent and/or other concentrations of guanidine thiocyanate are also within the scope of the invention.

Preferably the invention provides a method as described herein, wherein a first part of said homogenate is treated with said chaotropic agent and leaving a second part of said homogenate untreated with chaotropic agent and comparing the results obtained from said first and said second part. This results in the presence of an internal control of each sample and hence, this minimizes the risk of false results due to bad or incomplete homogenization cq. digestion. The obtained value for the non-treated sample (N) reflects all contributions to the signal that originate from incomplete homogenization and/or digestion, but also from artefacts from the immunochemical detection procedure (contributions to the end signal like non-specific binding of the conjugate). A sample treated with a chaotropic agent (T) shows a remarkable increase in signal compared to the non-treated sample (N) whereas a negative sample shows equal values for T and N.

The T/N value may be used to determine the status of the homogenate:

$$T/N \approx\leq 1 \text{ for negative samples}$$

$$T/N \gg 1 \text{ for positive samples}$$

Alternatively the T-N value may be used to discriminate between negative samples and positive samples:

$$T-N \approx 0 \text{ for negative samples}$$

$$T-N \gg 0 \text{ for positive samples}$$

The T/N x T-N values may also be used to discriminate between negative and positive samples:

$$T/N \times T\text{-}N \approx 0 \text{ for negative samples}$$

$$T/N \times T\text{-}N \gg 0 \text{ for positive samples}$$

However, in case one would not want to use a control for the chaotropic treatment (hence leave out the N samples), for example when large amount of samples are assayed, the T-values may be used to discriminate between negative and positive samples:

$$T \leq \text{cut-off for negative samples}$$

$$T > \text{cut-off for positive samples}$$

Optionally, controls are added to the method to check for proper digestion, and for obtaining T/N, T-N, or T-values for low positive samples.

In yet another preferred embodiment, the protease used in a method according to the invention is proteinase K. Preferably, a homogenate is diluted before protease treatment and even more preferably a homogenate is typically diluted 11-fold prior to a 3-fold dilution in a Proteinase K solution. Proteolytic digestion preferably takes place at approximately 50°C during approximately 30 minutes. A control that checks for proper digestion is optionally added. Even more preferably, after digestion with a protease, the samples are transferred to a carrier (for example a PVDF filter plate).

In a preferred embodiment, the invention provides a method for determining whether an aberrant prion protein is present or absent in a mammalian sample comprising the steps of preparing in an extraction buffer that comprises a surfactant and a detergent a homogenate from said sample, incubating said homogenate in diluted extraction buffer with a protease, incubating with a chaotropic agent and at least one washing buffer and detecting said aberrant prion protein, wherein said sample is obtained from brain stem.

Because of its simplicity and speed, a method according to the invention particularly lends itself to mass screening purposes of e.g. post-mortem tissues in the slaughter-line of ruminants such as cattle and sheep, but it is equally suitable in testing samples derived from other ruminants (for example elk or deer or experimental animals). In the human field the method is used for e.g. screening lymphoid tissues and blood-derived products. Essentially, samples from all tissues, body fluids (e.g. blood, liquor) and faeces from all kinds of animals (for example but not limited to humans) may be used. Furthermore, the sample may be fresh or may have been frozen (for example -20°C to -70°C). As disclosed herein within the experimental part, long term (frozen) storage does not affect the outcome of a method according to the invention.

The sampling of the proper region of the brain stem (Obex region) has become a standard procedure in BSE-tests (for example samples automatically taken from the brain at the time that the heads are cut off from the slaughter-animals' trunk) and hence no further details on this matter are provided. Preferably, the method according to the invention is used as a post-mortem test on brain stems from cattle older than 24 months.

The method is also used in preclinical stages during the development of scrapie, since tonsils which can be taken from the living animal, are proven to be an indicator tissue for preclinical scrapie and to contain PrP[Sc] (Schreuder et al., 1998). Samples from tonsils are homogenised in a similar way as described herein for samples from brain stems and hence a method according to the invention is also used on samples from tonsils.

When one would like to perform the method according to the invention on a fluid sample (for example blood or urine or an already prepared homogenate) it is clear that the homogenization step does not have to be performed. Hence, the scope of the invention includes a method for determining whether an aberrant prion protein is present or absent in a mammalian fluid sample comprising the steps of bringing said sample in extraction buffer (for example by dialysis) or diluting said sample in extraction buffer, incubating said fluid sample in diluted extraction buffer with a protease, incubating with a chaotropic agent and at least one washing buffer and detecting said aberrant prion protein. It is clear that in the cases of an (already) fluid sample, the time for completing the method according to the invention will be further reduced when compared to the time mentioned in Figure 7.

With regard to the detection steps of the presently claimed method, a compound capable of recognizing said aberrant prion protein preferably is an antibody directed against a protease (proteinase K) resistant part of the aberrant prion protein. An example of a suitable antibody (1 E5) is provided herein within the experimental part. Preferably, the antibody is coupled to a means that facilitate detection, for example horseradish peroxidase. Preferably, the for example, anti-PrP monoclonal 1 E5 conjugated to horseradish peroxidase in TBST+ is allowed to incubate for (30 minutes) at ambient temperature, followed by (3) washing steps (3 minutes incubation each) with TBST+. The plates, filtered to dryness, are inserted into a chemiluminometer (with injection pumps). A chemiluminescence substrate is added (optionally by a machine) and after a short time the generated photons are counted during a fixed time by the luminometer. Raw data are send to a computer for data reduction. It is clear that a functional equivalent and/or a functional fragment of the mentioned antibody is also included herein. Such a functional equivalent and/or functional fragment preferably has the same specificity however, does not necessarily have to perform in the same amount. A functional fragment is for example obtained by deleting certain parts of the antibody. A functional equivalent is for example obtained by inducing an antibody response to a similar antigen and then testing whether these new antibodies compete with the 1 E5 monoclonal.

Although it is clear that the order of the method steps as described above may be adapted according to the user's requirements, in a preferred embodiment the order of steps is:

- preparing a homogenate from said sample with an extraction buffer that comprises a surfactant and a detergent,
- incubating said homogenate with a protease
- applying the protease treated homogenate to a carrier
- incubating part of the protease treated homogenate with a chaotropic agent and optionally leaving a second part of the protease treated homogenate untreated
- subjecting the treated homogenate to at least one washing step
- incubating said carrier with a blocking buffer
- incubating said carrier with a compound capable of recognizing said aberrant prion protein
- visualising said compound.

Preferably, said surfactant and detergent are anyone of the suitable components as mentioned herein and even more preferably said surfactant is NaDOC and said detergent is Triton (preferably Triton X-100). More preferably, said blocking buffer comprises PVA (for example PVA 30-70 kDa) and BSA (for example fractionV) in trisbuffer/tween (TBST) and even more preferably, said blocking buffer comprises 1% PVA and 0.5% BSA in TBST. However, other suitable combination are 0.5% SM and 2% PVA or 1% SM and 0.5% PVA or 1% SM and 2% PVA or 2% SM and 1% PVA. The specific order of steps, in addition to an extraction buffer that comprises NaDOC and Triton and a blocking buffer that comprises PVA and BSA, further adds to the desired characteristics of a TSE diagnostic assay, for example high sensitivity and specificity. And hence, the method according to the invention also provides a method for determining whether an aberrant prion protein is present that reduces the risk of scoring a false-positive and/or false negative test and/or is completed with a strongly reduced analysis time. Preferably the time to come to a complete analysis is less than 6 hours, more preferably, less than 5 hours, even more preferably less than 4 hours and most preferred the analysis is completed in approximately 3.5 hours. As already outlined, the analysis time is even further reduces when a fluid sample is used.

The experimental part furthermore discloses preferred amounts of volumes that may be used as guidance for the method of the invention.

In a preferred embodiment, the method according to the invention is adapted such that most of the steps are performed automatically. For example, the digestion procedure and the detection procedure may be fully automated on a robotic system. A robot typically is adjusted such that it accepts a deep-well collection plate as input and processes the steps mentioned in the digestion (protease and chaotropic agent treatment)/detection procedure. Finally the PVDF filter plates are inserted into the chemiluminometer. An example of a suitable robot is any ELISA-robot that is capable of applying a vacuum pressure to the bottom of an ELISA plate and, on the same platform, perform a digestion at 50°C. Optionally, provisions may be made for two software driven vacuum manifolds, one plate position for the pipetting tips, one plate position for the reagents (i.e. chaotropic solution, PBS, 1 E5 conjugate solution, washing solution), one plate position for the 50 °C heating block and one position for the deep-well collection plate.

Besides the already above-mentioned advantages, a method according to the invention furthermore results in less produced waste material. All waste material obtained from a TSE diagnostic assay might be possible contaminated and hence must be separately collected and destroyed. Some of the presently used methods produce up to 1.5 to 8 litres of (possible) contaminated waste for the analysis of 100 samples. The method according to the invention typically produces less then 0.5 litres of waste for the same amount of samples. Therefore, the invention further more provides a method for determining whether an aberrant prion protein is present in a sample which method results in a strongly reduced amount of waste.

In yet another embodiment the invention provides a kit comprising the means for performing a method as described herein. Preferably, said kit comprises at least a chaotropic agent and an extraction buffer that comprises a surfactant and a detergent, for example NaDOC and Triton. In a preferred embodiment, said kit further comprises a blocking buffer that comprises polyvinylalcohol and bovine serum albumin in trisbuffer/tween.

Preferably, said kit is a so-called ready for use kit that comprises all means for performing a method as described herein, except the methanol.

The invention will now be illustrated by means of the following, nonlimiting examples.

**EXPERIMENTAL PART MATERIALS & METHODS**

**Reagents & buffers**

- PBS: 10 mM $NaH_2PO_4$, 10 mM $K_2HPO_4.2H_2O$ and 150 mM NaCl
- extraction buffer: 0.5% NaDOC (sodium deoxycholate), 0.5 % Triton X100 in PBS; NaDOC (Merck, article number 6504, 250 gram), Triton X100 (BDH article number 30632, 500 ml ); 0.5 gram NaDOC + 0.5 ml Triton X100 in PBS13 (final volume 100 ml)
- dilutionbuffer: extractionbuffer 10x diluted in PBS
- SQ: Super Q water
- Proteinase K (Merck article number 1.24568, 100 mg); 11 mg proteinase K (20 units/mg lyophilisate) in 1 ml 50 mM Tris-HCl buffer + 1 mM $CaCl_2$, pH 8.0; store concentrate at -20°C in appropriate portions
- 1M $CaCl_2$: dissolve 0.147 gram $CaCl_2$ $2H_2O$ in SQ (final volume: 10 ml)
- 50 mM Tris-HCl: dissolve 0.61 gram Tris in 100 ml SQ; adjust pH to 8.0 with 4M HCl ($\pm$ 1 ml)
- fresh Proteinase K solution: dilute above-mentioned concentrate 20x in PBS
- Trisbuffer: 50 mM Tris + 150 mM NaCl and adjust pH to 7.5
- TBS: Trisbuffer without Tween
- TBST: Trisbuffer + 0.05% Tween 20
- blocking buffer: 1% PVA + 0.5% BSA dissolved in TBST
- chaotropic agent: 4 M Guanidine thiocyanate (GdnSCN; Simga, article number g-9277, 500 gram); to 2.364 gram SQ is added to a volume of 5 ml
- PVDF-filter plates: customised Whatman PVDF filterplates (Whatman 7700-3356 or 7700-4356); alternatives: Millipore Multiscreen (MAGV S22 10, MAHV S45 10, MADV S65 and MABV S12 10) or Pall AcroWell 96 plates or PVDF plates from Corning or Innovative Microplates
- chemiluminescence mix: the standard chemiluminescence buffer of BioFX (CHMI-0060-2C or CHMM-0060-2C) is diluted 50x in PBS
- antibodies are prepared by standard proceedings; in short PrP knockout mice were immunized with huPrP-MBP and boPrP; approximately 4 immunisations in the presence of Freund's adjuvant were performed; three days before fusion a fifth injection was provided; on the day of fusion 50 µl 0.2% Bordetella antigen was provided intravenously; fusion with SP2/0 cells; screening was performed with boPrP coated on a polystyreen microtiterplate.

In a preferred embodiment, the kit according to the invention comprises all mentioned buffers and reagents in a ready-for-use mode and the extraction buffer is provided as a 5x concentrate.

**BSE test**

In one of the embodiments, the method according to the invention is essentially performed in the following way:

1. Add to a Whatman (PVDF filter) plate 50 µl methanol and vacuum-filtrate;
2. Add 2 x 200 µl water en vacuum-filtrate;
3. Dilute the (brain stem) homogenate 1/6 in dilutionbuffer (40 µl homogenate in 200 µl buffer);
4. Dilute the Proteinase K (PK) substrate by adding 50 µl Proteinase concentrate to 6600 µl dilutionbuffer;
5. Add 80 µl diluted PK to each well of the incubation plate; if a control to the PK digestion is desired 80 µl dilutionbuffer is added
6. Incubate the plate for 30 min at 50 °C;
7. Add 40 µl of each sample to the (N and/or T) part of the Whatman plate;
8. Incubate 30 minutes at room temperature and vacuum-filtrate the plate;
9. Incubate the T part for 10 minutes with 50 µl chaotropic agent and the N part with 50 µl PBS;
10. Vacuum-filtrate the plate;
11. Add 2 x 200 µl PBS en vacuum-filtrate;
12. Add 2 x 200 µl blockingbuffer (incubate for 3 minutes each) and vacuum-filtrate;
13. Resuspend the 1E5 Mab;
14. Add to each well 50 µl diluted 1E5 Mab solution and incubate 30 minutes at room temperature;

15. Vacuum filtrate the plate;

16. Add 3 x 200 µl blockingbuffer; incubate for 3 minutes and vacuum filtrate;

17. Mix the two components that make up the ready-for-use chemiluminescence substrate;

18. Add to each well 50 µl chemiluminescence substrate;

19. Measurements are made with help of a chemiluminometer; 0.5 seconds each well.

**Preparation of 1+1 CNS macerates for proficiency testing of BSE-laboratories**

Material:

- CNS-tissue of TSE-infected animals
- CNS-tissue of normal individuals
- Pure water (Distilled or Milli-Q quality)

Equipment:

- Stainless steel sieve (fine-meshed, round-bottomed, e.g. Eva Trio)
- Stainless steel spoon (hollow, half-spherical)
- Cylindrical glass beaker (250 ml)
- Scissors (one blunt end)
- Adjustable pipettes (0.1 - 5 ml)
- Disposable pipettes
- Balance
- Vortex-mixer

Procedure (small scale):

1. Weigh out 5 - 10 g of CNS-tissue and place into the sieve (6.5 cm diameter). Transfer the corresponding ml's of pure water into a clean tube.
2. Cut the tissue into small pieces using the scissors with the blunt leg towards the sieve
3. Produce a smear of tissue by grinding with the spoon (3.8 cm) in a rotating motion towards the bottom of the sieve
4. Add 1 ml of water to the hollow upper side of the spoon. Continue with rotating movements while adding small volumes of water from the spoon to the smear. The tissue will take up the water and swell
5. Add more portions of water slowly by way of the spoon. Use the edge of the spoon to scrape the tissue smear towards the bottom of the sieve.
6. When all the water has been added, continue to press the remaining smear through the sieve. Additional tissue can be rescued with the spoon from the underside of the sieve.
7. Use the disposable pipettes (suction and aspiration) for mixing the material at the bottom of the glass beaker. Transfer with a pipette to a pre-balanced 15-ml or 50-ml tube
8. Weigh out the macerate. The recovery should be 75 - 90 %.
9. Vortex the content vigorously to obtain uniform distribution of $PrP^{SC}$.

Procedure (larger scale):

Up to 40 grammes of CNS-tissue can be handled in larger sieves and with a larger spoon. It is advisable in this case to add tissue in smaller portions (10 - 15 g).

**EXPERIMENTAL PART RESULTS**

**Details of initial evaluation**

In an initial evaluation study 8 BSE positive samples (from frozen stocks) and 84 freshly prepared brain stem Obex homogenates (which were all tested negative in the Prionics Western Blot assay) were tested according to the method of the invention as described herein. The positive samples ranged in chemiluminescence relative light units (rlu) from $4910 \le$ T-N $\le 41770$. The negative samples ranged from $-90 \le$ T-N $\le 130$ with an average of 8 rlu, and a SD of 47 rlu resulting in a gap of 103 times the SD between the highest negative and the lowest positive value. This is indicative for the fact that a very low level of sensitivity has been obtained.

**Sensitivity and specificity under field conditions**

The initial evaluation (see above) shows 100% sensitivity and 100% specificity, with a very low level of detection of 474 rlu (average + 10*SD). On a western blot the monoclonal used in the method (designated monoclonal 1 E5) shows full cross reactivity with PrP$^{Sc}$ from sheep.

**Results obtained from other TSE: scrapie (sheep)**

An initial evaluation with 14 negative and 4 positive Scrapie samples shows 100% sensitivity and 100% specificity. The positive samples ranged in chemiluminescence relative light units (rlu) from $39700 \le$ T-N $\le 286000$. The negative samples ranged from $-770 \le$ T-N $\le 150$ with an average of 140 rlu, and a SD of 425 rlu resulting in a gap of 93 times the SD between the highest negative and the lowest positive value. This is indicative for the fact that a very low level of sensitivity can be obtained.

**Results obtained from other TSE: chronic wasting disease (deer/elk)**

An initial evaluation with 24 negative and 24 non-digested negative samples from brain stem homogenates for the White Spotted Deer shows 100% sensitivity and 100% "specificity". The non-digested "positive" samples ranged in chemiluminescence relative light units (rlu) from $99200 \le$ T-N $\le 120000$. The negative samples ranged from $-80 \le$ T-N $\le 90$ with an average of 20 rlu, and a SD of 90 rlu resulting in a gap of 1101 times the SD between the highest negative and the lowest "positive value".

**Reproducibility of results within and between antibody batches stability of analyte in sample matrix, analytical standards**

In an early stage of the test development when different batches of the antibody 1 E5 were used in a double determinant assay on photographic film or with a condensing substrate on PVDF membrane the same dilutions of the monoclonal from different batches showed comparable intensities. The method of the invention however uses the 1 E5 monoclonal conjugated to HRP (standard conjugation protocol). Of this 1 E5-HRP conjugate numerous batches have been prepared to date.

The within antibody conjugate batch reproducibility is acceptable and within 10%. Standard ready for use dilutions are prepared in StabilZym® , showing excellent stability.

No studies have been undertaken yet to assess the stability of homogenates in the homogenization buffer. However, since in general negative homogenates always are at T-N values of around 0 (whether frozen or fresh) the analyte seems stable in the homogenization matrix. Most of the developmental work of the method has been carried out on frozen samples (positive and negative). The positive samples show a strong tendency to become less positive over time, albeit non of the positive samples reached the detection limit yet (some of the positive samples have been in our freezer for more than 3 years now).

The analytical standards/controls (dilutions of rec-PrP$^C$) are prepared fresh for each experiment.

**Stage of the disease as from when the test is useful**

To date only brain stems from cattle older than 24 months have been tested. The method for testing the presence of PrP$^{Sc}$ is designed to operate as a post-mortem test on brain stems from cattle older than 24 months. However, evidenced by the large gap between the lowest positive sample and the highest negative sample there is plenty of room to detect lower positive samples (perhaps even pre-clinical samples).

**Detection of aberrant prion protein in BSE**

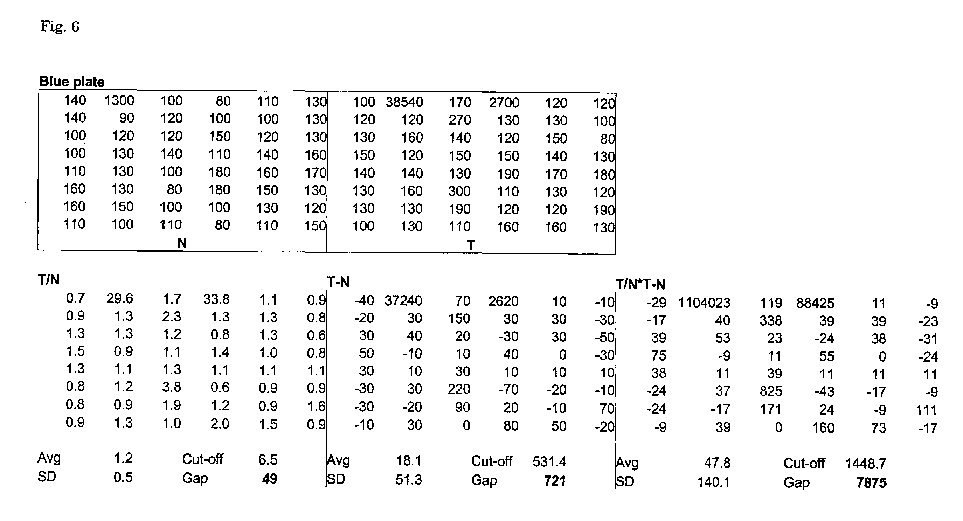

The method as described herein was applied to fresh brain samples from cattle. The method included two positive controls. This experiment has been performed with colour-coded PVDF-filter plates. The obtained experimental results are determined on basis of the T- value, the T-N value and the T-N x T/N value and are depicted in Figure 6.

**The effect of blocking buffer composition on the amount of false-positives and false-negatives**

An experiment was performed to determine the effect of the blocking buffer composition on the amount of false-positives and false-negatives:

| TBST comprising | Positives | Negatives |
|---|---|---|
| BSA 0,5%, PVA 1% | 8/8 | 16/16 |
| BSA 0,5%, PVA 2% | 8/8 | 15/16, 1 false |
| BSA 0,25%, PVA 1% | 8/8 | 15/16, 1 false |
| BSA 0,25%, PVA 2% | 8/8 | 15/16, 1 false |
| SM 0,5%, PVA 0,5% | 8/8 | 8/8, 2 high |
| SM 0,5%, PVA 1% | 8/8 | 6/8, 2 false |
| SM 0,5%, PVA 2% | 8/8 | 8/8 |
| SM 1%, PVA 0,5% | 8/8 | 8/8 |
| SM 1%, PVA 1% | 8/8 | 6/8, 2 false |
| SM 1%, PVA 2% | 8/8 | 8/8 |
| SM 2%, PVA 0,5% | 7/8, 1 false | 7/8, 1 false |
| SM 2%, PVA 1% | 8/8 | 8/8 |
| SM 2%, PVA 2% | 8/8 | 6/8, 2 false |

**Experiments performed on fluid samples**

Ten 1 ml EDTA-blood samples were centrifuged at 3000 g for 10 minutes and the remaining plasma was spiked with 120 ng rec-PrP. An initial evaluation with 10 negative and 10 non-digested negative Prp spiked plasma samples shows 100% sensitivity and 100% "specificity". The non-digested "positive" samples ranged in chemiluminescence relative light units (rlu) from $118000 \leq T\text{-}N \leq 124000$. The negative samples ranged from $-35 \leq T\text{-}N \leq 68$ with an average of 19 rlu, and a SD of 58 rlu resulting in a gap of 2033 times the SD between the highest negative and the lowest "positive value".

Ten 1 ml urine samples were spiked with 120 ng rec-PrP. An initial evaluation with 10 negative and 10 non-digested negative Prp spiked urine samples show 100% sensitivity and 100% "specificity". The non-digested "positive" samples ranged in chemiluminescence relative light units (rlu) from $73000 \leq T\text{-}N \leq 79000$. The negative samples ranged from $-43 \leq T\text{-}N \leq 80$ with an average of 28 rlu, and a SD of 68 rlu resulting in a gap of 1072 times the SD between the highest negative and the lowest "positive value".

**DESCRIPTION OF FIGURES**

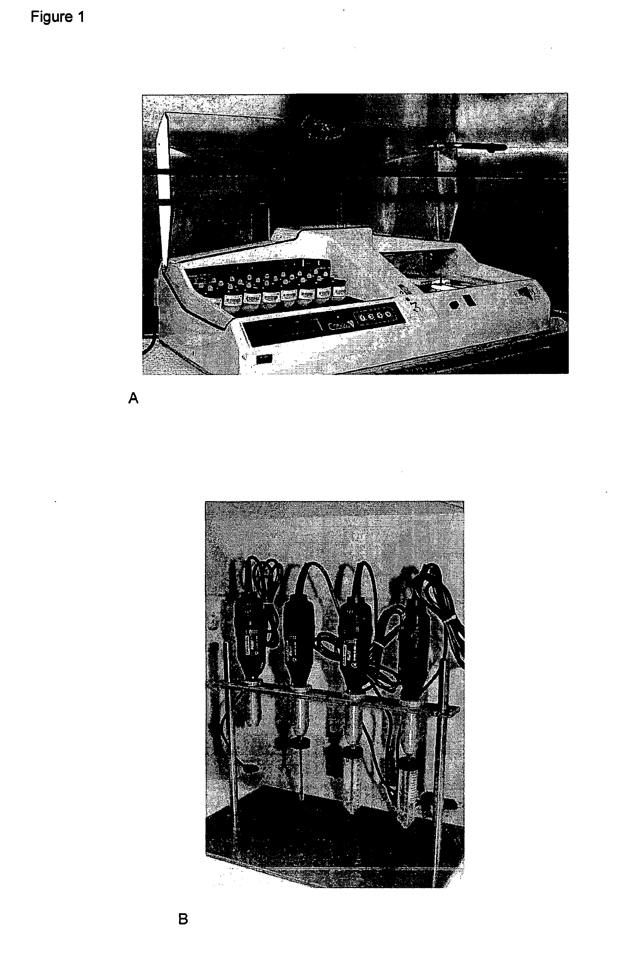

**Figure 1.** Pictures of FastH (A) and other homogenisation devices (Omni, Dremel, UltraTurrax, B).

**Figure 2.** Transfer of homogenates to the deep-well collection plate; row A (marked, green zone) is left open for controls to be added later on in the procedure.

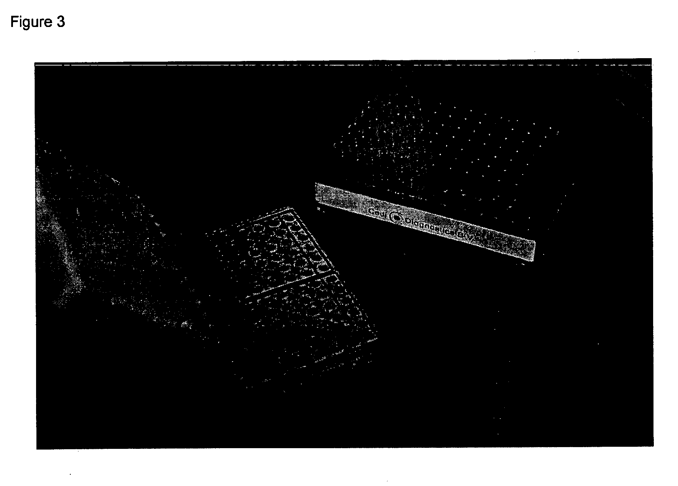

**Figure 3.** Transfer of homogenate from the deep-well plate to the incubation plate.

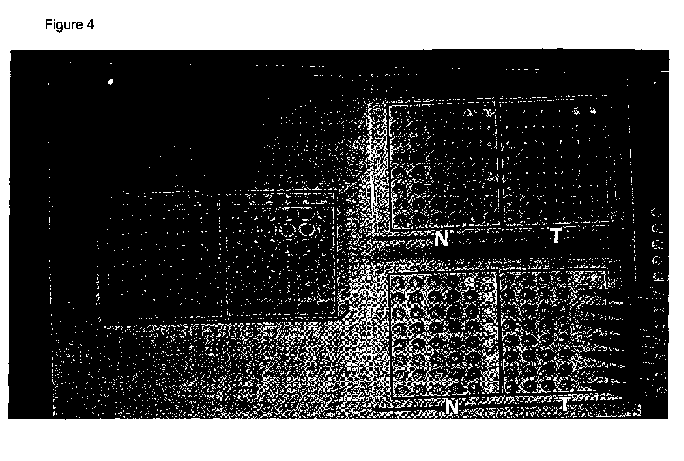

**Figure 4.** Transfer from the incubation plate top the 96-well PVDF filter plates (the blue part is transferred in duplicate to the blue plate, the red part is transferred in duplicate to the red plate).

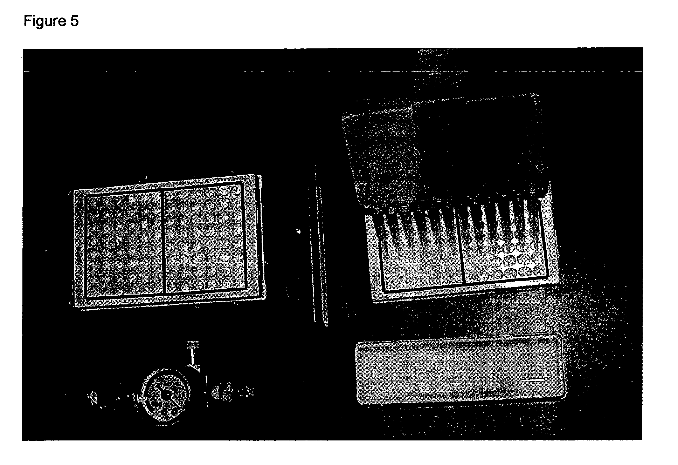

**Figure 5.** Detection procedure is carried out with fast and efficient washing by filtration on a vacuum manifold.

**Figure 6.** Example of obtained results.

**Figure 7.** Comparison of different TSE tests

**REFERENCES**

- Belt, P.B.F.M., I. Muileman, B.E.C. Schreuder, J. Bos-de Ruijter, A.L.J. Gielkens & M.A. Smits (1995). Identification of five allelic variants of the sheep PrP gene and their association with natural scrapie. J. Gen. Virol. 76:509-517.
- Billeter, M. R. Riek, G. Wider, S. Hornemann, R. Glockshuber & K. Wüthrich. (1997). Prion protein NMR structure and species barrier for prion diseases. Proc. Natl. Acad. Sci. USA 94:7281-7285.
- Bossers, A., B.E.C. Schreuder, I.H. Muileman, P.B.G.M. Belt & M.A. Smits (1996). PrP genotype contributes to determining survival times of sheep with natural scrapie. J. Gen. Virol. 77:2669-2673.
- Bruce, M.E., & H. Fraser (1991). Scrapie strain variation and implications. In: Chesebro B. (ed.). Transmissible spongiform encephalopathies. Current topics Microbiol. Immunol. 172:125-138.
- Bruce, M.E., R.G. Will, J.W. Ironside, I. McConnell, D. Drummond, A. Suttle, L. McCardle, A. Chree, J. Hope, C. Birkett, S. Cousens, H. Fraser & C.J. Bostock (1997). Transmissions to mice indicate that 'new variant' CJD is caused by the BSE agent. Nature 389:498-501.
- Caughey, B. & G.J. Raymond (1991). The scrapie-associated form of PrP is formed from a cell surface precursor that is both protease- and phospholipase-sensitive. J. Biol. Chem. 266:18217-18223.
- Collinge, J., K.C.L. Sidle, J. Meads, J. Ironside & A.F. Hill (1996). Molecular analysis of prion strain variation and the aetiology of 'new variant' CJD. Nature 383:685-690.
- Donne, D.G., J.H. Viles, D. Groth, I. Mehlhorn, T.L. James, F.E. Cohen, S.B. Prusiner, P.E. Wright & H.J. Dyson (1997). Structure of the recombinant full-length hamster prion protein PrP(29-131): the N terminus is highly flexible. Proc. Natl. Acad. Sci. U.S.A. 94: 13452-13457.
- Foster, J., J. Hope & H. Fraser (1993). Transmission of bovine spongiform encephalopathy to sheep and goats. Vet. Rec. 133:339-341.
- Gibbs, C.J. Jr., J. Safar, M. Ceroni, A. DiMartino, W.W. Clark & J.L. Hourrigan (1990). Experimental transmission of scrapie to cattle. Lancet 335:1275.
- Hourrigan, J.L. (1990). Experimentally induced bovine spongiform encephalopathy in cattle in Mission, Tex, and the control of scrapie. J. Am. Vet. Med. Assoc. 196:1678-1679.
- Hunter, N., W. Goldmann, G. Smith & J. Hope (1994). The association of a codon 136 PrP gene variant with the occurrence of natural scrapie. Arch. of Virol. 137:171-177.
- Korth, C., B. Stierli, P. Streit, M. Moser, O. Schaller, R. Fischer, W. Schulz-Schaeffer, H. Kretzschmar, A. Raeber, U. Braun, F. Ehrensperger, S. Hornemann, R. Glockshuber, R. Riek, M. Billeter, K. Wüthrich & B. Oesch (1997). Prion PrPSc-specific epitope defined by a monoclonal antibody. Nature 390:74-77.
- Laplanche, J.L., J. Chatelain, D. Westaway, S. Thomas, M. Dussausy, J. Brugere-Picoux & J.M. Launay (1993). PrP polymorphism associated with natural scrapie discovered by denaturing gradient gel electrophoresis. Genomics 15:30-37.
- Pan, K.-M., Baldwin, M., Nguyen, J., Gasset, M., Serban, A., Groth, D., Mehlhorn, I., Huang, Z., Fletterick, R.J., Cohen, F.E. & S.B. Prusiner (1993). Conversion of alpha-helices into beta-sheets features in the formation of the scrapie prion proteins. Proc. Natl. Acad. Sci. U.S.A. 90:10962-10966.
- Pattison, I.H. (1988). Fifty years with scrapie: a personal reminiscence. Vet. Rec. 123:661-666.
- Prusiner, S.B., M.R. Scott, S.J. DeArmond & F.E. Cohen (1998). Prion protein biology. Cell 93:337-348.
- Riek, R., S. Hornemann, G. Wider, M. Billeter, R. Glockshuber & K. Wüthrich(1996). NMR-structure of the mouse prion protein domain PrP(121-231). Nature 382:180-182.
- Riek, R., S. Hornemann, G. Wider, R. Glockshuber & K. Wüthrich (1997) NMR characterization of the full-length recombinant murine prion protein, mPrP(23-231). FEBS Lett. 413: 282-288.
- Safar,.J, H. Wille, V. Itri, D. Groth, H. Serban, M.Torchia, F.E. Cohen & S.B. Prusiner 1998). Eight prion strains have PrPSc molecules with different conformations. Nature Medicine 10:1157-1165.
- Schatzl, H.M., M. Da Costa, L. Taylor, F.E. Cohen & S.B. Prusiner (1995). Prion protein gene variation among primates. J. Mol. Biol. 245:362-347.
- Schreuder, B.E.C., L.J.M. van Keulen, M.E.W. Vromans, J.P.M. Langeveld & M.A. Smits (1998). Tonsillar biopsy and PrPSc detection in the preclinical diagnosis of scrapie. Vet. Rec. 142:564-568.
- Wilesmith, J.W., J.B.M. Ryan & M.J. Atkinson (1991). Bovine spongiform encephalopathy: epidemiological studies on the origin. Vet. Rec. 128:199-203.
- Will, R.G., J.W. Ironside, M. Zeidler, S.N. Cousens, K. Estibeiro, A. Alperovitch, S. Poser, M. Pocchiari, A. Hofman & P.G. Smith (1996). A new variant of Creutzfeldt-Jakob disease in the UK. Lancet 347:921-925.

**Claims**

1. A method for determining whether an aberrant prion protein is present or absent in a mammalian sample comprising the steps of preparing a homogenate from said sample with an extraction buffer that comprises a surfactant and a detergent, incubating said homogenate in diluted extraction buffer with a protease, incubating with a chaotropic agent and at least one washing buffer and detecting said aberrant prion protein.

2. A method according to claim 1, wherein said surfactant is sodium deoxycholate (NaDOC).

3. A method according to claim 1 or 2, wherein said detergent is Triton X100.

4. A method according to any one of claims 1 to 3, further comprising applying said homogenate to a carrier.

5. A method according to any one of claims 1 to 4, wherein said aberrant prion protein is detected in an immunoassay.

6. A method according to claim 4 or 5, wherein detection of said aberrant prion protein comprises the steps of incubating said carrier with blocking buffer, a compound capable of recognizing said aberrant prion protein and visualising said compound.

7. A method according to any one of claims 1 to 6, wherein said chaotropic agent is guanidine thiocyanate.

8. A method according to claim 6 or 7, wherein said blocking buffer comprises polyvinylalcohol and bovine serum albumine in trisbuffer/tween.

9. A method according to any one of claims 4 to 8, wherein said homogenate is applied to a carrier after protease incubation.

10. A method according to any one of claims 1 to 9, wherein said protease is proteinase K.

11. A method according to any one of claims 4 to 10, wherein said carrier is a filter.

12. A method according to claim 11, wherein said filter is a PVDF filter.

13. A method according to any one of claims 1 to 12, wherein said sample is obtained from brain stem or wherein said sample is obtained from a tonsil or wherein said sample is blood.

14. A method according to any one of claims 1 to 13, wherein a first part of said homogenate is treated with said chaotropic agent and leaving a second part of said homogenate untreated with chaotropic agent and comparing the results obtained from said first and said second part.

15. A method according to anyone of claims 6 to 14, wherein said compound capable of recognizing said aberrant prion protein is an antibody directed against a protease resistant part of the aberrant prion protein.

16. A method according to claim 15, wherein said protease is proteinase K.

17. A method according to any one of claims 6 to 16, wherein the order of steps is:

   - preparing a homogenate from said sample with an extraction buffer that comprises a surfactant and a detergent
   - incubating said homogenate with a protease
   - applying the protease treated homogenate to a carrier
   - incubating part of the protease treated homogenate with a chaotropic agent and optionally leaving a second part of the protease treated homogenate untreated
   - subjecting the treated homogenate to at least one washing step
   - incubating said carrier with a blocking buffer
   - incubating said carrier with a compound capable of recognizing said aberrant prion protein
   - visualising said compound.

18. A method according to claim 17, wherein said surfactant is NaDOC.

**19.** A method according to claim 17 or 18, wherein said detergent is Triton.

**20.** A kit comprising the means for performing a method according to any one of claims 1 to 19.

**21.** A kit comprising at least a chaotropic agent and an extraction buffer that comprises a surfactant and a detergent.

**22.** A kit according to claim 21, wherein said surfactant is NaDOC and said detergent is Triton.

**23.** A kit according to claim 21 or 22, further comprising a blocking buffer that comprises polyvinylalcohol and bovine serum albumine in trisbuffer/tween.

**24.** A kit according to any one of claims 1 to 23, further comprises a colour-coded carrier.

Figure 1

A

B

Figure 2

Figure 3

Figure 4

Figure 5

Fig. 6

**Blue plate**

| 140 | 1300 | 100 | 80 | 110 | 130 | 100 | 38540 | 170 | 2700 | 120 | 120 |
|---|---|---|---|---|---|---|---|---|---|---|---|
| 140 | 90 | 120 | 100 | 100 | 130 | 120 | 120 | 270 | 130 | 130 | 100 |
| 100 | 120 | 120 | 150 | 120 | 130 | 130 | 160 | 140 | 120 | 150 | 80 |
| 100 | 130 | 140 | 110 | 140 | 160 | 150 | 120 | 150 | 150 | 140 | 130 |
| 110 | 130 | 100 | 180 | 160 | 170 | 140 | 140 | 130 | 190 | 170 | 180 |
| 160 | 130 | 80 | 180 | 150 | 130 | 130 | 160 | 300 | 110 | 130 | 120 |
| 160 | 150 | 100 | 100 | 130 | 120 | 130 | 130 | 190 | 120 | 120 | 190 |
| 110 | 100 | 110 | 80 | 110 | 150 | 100 | 130 | 110 | 160 | 160 | 130 |
| | | **N** | | | | | | **T** | | | |

**T/N**

| 0.7 | 29.6 | 1.7 | 33.8 | 1.1 | 0.9 |
|---|---|---|---|---|---|
| 0.9 | 1.3 | 2.3 | 1.3 | 1.3 | 0.8 |
| 1.3 | 1.3 | 1.2 | 0.8 | 1.3 | 0.6 |
| 1.5 | 0.9 | 1.1 | 1.4 | 1.0 | 0.8 |
| 1.3 | 1.1 | 1.3 | 1.1 | 1.1 | 1.1 |
| 0.8 | 1.2 | 3.8 | 0.6 | 0.9 | 0.9 |
| 0.8 | 0.9 | 1.9 | 1.2 | 0.9 | 1.6 |
| 0.9 | 1.3 | 1.0 | 2.0 | 1.5 | 0.9 |

| Avg | 1.2 | Cut-off | 6.5 |
|---|---|---|---|
| SD | 0.5 | Gap | **49** |

**T-N**

| -40 | 37240 | 70 | 2620 | 10 | -10 |
|---|---|---|---|---|---|
| -20 | 30 | 150 | 30 | 30 | -30 |
| 30 | 40 | 20 | -30 | 30 | -50 |
| 50 | -10 | 10 | 40 | 0 | -30 |
| 30 | 10 | 30 | 10 | 10 | 10 |
| -30 | 30 | 220 | -70 | -20 | -10 |
| -30 | -20 | 90 | 20 | -10 | 70 |
| -10 | 30 | 0 | 80 | 50 | -20 |

| Avg | 18.1 | Cut-off | 531.4 |
|---|---|---|---|
| SD | 51.3 | Gap | **721** |

**T/N\*T-N**

| -29 | 1104023 | 119 | 88425 | 11 | -9 |
|---|---|---|---|---|---|
| -17 | 40 | 338 | 39 | 39 | -23 |
| 39 | 53 | 23 | -24 | 38 | -31 |
| 75 | -9 | 11 | 55 | 0 | -24 |
| 38 | 11 | 39 | 11 | 11 | 11 |
| -24 | 37 | 825 | -43 | -17 | -9 |
| -24 | -17 | 171 | 24 | -9 | 111 |
| -9 | 39 | 0 | 160 | 73 | -17 |

| Avg | 47.8 | Cut-off | 1448.7 |
|---|---|---|---|
| SD | 140.1 | Gap | **7875** |

Figure 7

The Prionics WB, BioRad Platelia, Enfer TSE and Ceditect® BSE test compared
(time in minutes for 100 samples)

| Step | Prionics WB | BioRad Platelia | Enfer TSE | invention |
|---|---|---|---|---|
| • Sample preparation | | | | |
|   - sample intake | 15 | 15 | 15 | 15 |
|   - Obex preparation | 45 | 60 | 45 | 45 |
|   - homogenization/centrifugation | 30 (h) | 55 (h/c) | 120 (h/c) | 30 (h) |
| • Digestion | 60 | 100 | 60 | 30 |
| • Western Blot / plate incubation | 120 (WB) | 0 | 0 | 30 (Pi) |
| • Detection of PrP$^{Sc}$ | 240 | 145 | 120 | 60 |
|     Total time (min) | 510 | 375 | 360 | 210 |
|     (hours) | 8½ | 6¾ | 6 | 3½ |

h = homogenization

c = centrifugation

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 03 001211 A (MAX PLANCK GESELLSCHAFT ;TATZELT JOERG (DE); HARTL ULRICH (DE); WI) 3 January 2003 (2003-01-03) * page 4 - page 9; examples 1-4 * | 1-24 | G01N33/68 C07K14/47 |
| X,D | WO 00 48003 A (BOSSERS ALEXANDER ;GARSSEN GERRIT JAN (NL); JACOBS JORG GUENTHER () 17 August 2000 (2000-08-17) | 1,4-7, 9-17,20, 21,23 | |
| Y | * page 18, paragraph 2 * * page 19, paragraph 2 * * page 20, paragraph 3 * * page 21 - page 28 * | 8,23 | |
| X | SERBAN D ET AL: "RAPID DETECTION OF CREUTZFELDT-JAKOB DISEASE AND SCRAPIE PRION PROTEINS" NEUROLOGY, LIPPINCOTT WILLIAMS & WILKINS, PHILADELPHIA, US, vol. 40, no. 1, January 1990 (1990-01), pages 110-117, XP001105804 ISSN: 0028-3878 * the whole document * | 1,2,4-7, 10,11, 14-16 | |
| Y | AUSUBEL ET AL.: "Current Protocols in Molecular Biology" 1999 , JOHN WILEY & SONS, INC. , NEW YORK XP002270597 * page 10.8.16 - page 10.8.20 * | 8,23 | **TECHNICAL FIELDS SEARCHED** (Int.Cl.7)<br><br>G01N<br>C07K |

The present search report has been drawn up for all claims

| Place of search | Date of completion of the search | Examiner |
|---|---|---|
| THE HAGUE | 20 February 2004 | Schalich, J |

EPO FORM 1503 03 82 (P04C01)

## ANNEX TO THE EUROPEAN SEARCH REPORT
## ON EUROPEAN PATENT APPLICATION NO.

EP 03 07 7783

This annex lists the patent family members relating to the patent documents cited in the above–mentioned European search report.
The members are as contained in the European Patent Office EDP file on
The European Patent Office is in no way liable for these particulars which are merely given for the purpose of information.

20-02-2004

| Patent document cited in search report | | Publication date | Patent family member(s) | | Publication date |
|---|---|---|---|---|---|
| WO 03001211 | A | 03-01-2003 | WO | 03001211 A1 | 03-01-2003 |
| WO 0048003 | A | 17-08-2000 | AU | 2580900 A | 29-08-2000 |
| | | | CA | 2364686 A1 | 17-08-2000 |
| | | | EP | 1151305 A1 | 07-11-2001 |
| | | | WO | 0048003 A1 | 17-08-2000 |

EPO FORM P0459

For more details about this annex : see Official Journal of the European Patent Office, No. 12/82